# EUROPEAN PATENT APPLICATION

(11) **EP 4 582 518 A1**
(43) Date of publication of application: **09.07.2025**
(21) Application number: 23860435.9
(22) Date of filing: 30.08.2023
(51) Int. Cl.: C10G 2/00, C07C 29/151, C07C 31/04, C10L 1/02

(54) **METHOD FOR PRODUCING LIQUID FUEL AND LIQUID FUEL SYNTHESIS SYSTEM**

(30) Priority: 01.09.2022 JP 2022139587
(71) Applicant: NGK Insulators, Ltd., Nagoya-shi, Aichi 467-8530 (JP)
(72) Inventor: IIDA, Kazuki, Nagoya-shi, Aichi 467-8530 (JP); MAEHARA, Sota, Nagoya-shi, Aichi 467-8530 (JP); ANDO, Junichi, Nagoya-shi, Aichi 467-8530 (JP); OKUMA, Yusuke, Nagoya-shi, Aichi 467-8530 (JP); KAN, Hirofumi, Nagoya-shi, Aichi 467-8530 (JP); SHIBAGAKI, Yukinari, Nagoya-shi, Aichi 467-8530 (JP); TAKAHASHI, Michio, Nagoya-shi, Aichi 467-8530 (JP)
(74) Representative: Müller-Boré & Partner Patentanwälte PartG mbB
(86) International application number: PCT/JP2023/031601
(87) International publication number: WO 2024/048673

(57) **Abstract**

A primary object of the present invention is to suppress a reduction in reaction efficiency near the gas inflow port of a reactor in a conversion reaction from a raw material gas containing a carbon oxide and hydrogen to a liquid fuel. A method of producing a liquid fuel according to an embodiment of the present invention includes: causing a raw material gas containing at least a carbon oxide and hydrogen to flow into a reactor storing a catalyst; and producing the liquid fuel from the raw material gas through a conversion reaction in the presence of the catalyst. The raw material gas further contains oxygen. The ratio of the concentration of carbon monoxide to the concentration of carbon dioxide in the carbon oxide is 0.9 or less.

## Description

### Technical Field

The present invention relates to a method of producing a liquid fuel and a liquid fuel synthesis system.

### Background Art

In recent years, the following has been proposed for the purpose of achieving a carbon neutral society: a carbon oxide, such as carbon monoxide or carbon dioxide, is grasped as a carbon resource, and is converted into a liquid fuel useful as an industrial basic raw material. In, for example, Patent Literature 1, there is a disclosure of a liquid fuel synthesis system that performs a conversion reaction from a raw material gas containing carbon dioxide (CO₂) and hydrogen (H₂) to methanol with a membrane reactor including a catalyst and a water vapor separation membrane.

### Citation List

### Patent Literature

[PTL 1] JP 2018-8940 A

### Summary of Invention

### Technical Problem

In such conversion reaction from a raw material gas containing a carbon oxide and hydrogen to a liquid fuel as described above, its reaction efficiency may reduce. Specifically, the above-mentioned conversion reaction is an exothermic reaction, and generated heat is effective in maintaining its reaction temperature. However, near the gas inflow port of a reactor, the generated heat is flowed toward the downstream side thereof by a gas flow, and hence a sufficiently high reaction temperature is not obtained. Thus, the reaction efficiency may reduce.

A primary object of the present invention is to suppress a reduction in reaction efficiency near the gas inflow port of a reactor in a conversion reaction from a raw material gas containing a carbon oxide and hydrogen to a liquid fuel.

### Solution to Problem

[1] According to an embodiment of the present invention, there is provided a method of producing a liquid fuel, including: causing a raw material gas containing at least a carbon oxide and hydrogen to flow into a reactor storing a catalyst; and producing the liquid fuel from the raw material gas through a conversion reaction in the presence of the catalyst, wherein the raw material gas further contains oxygen, and wherein a ratio of a concentration of carbon monoxide to a concentration of carbon dioxide in the carbon oxide is 0.9 or less.
[2] In the production method according to the above-mentioned item [1], a concentration of the oxygen in the raw material gas may be 0.02 vol% or more and 4 vol% or less.
[3] In the production method according to the above-mentioned item [1] or [2], a ratio of a concentration of the oxygen to a concentration of the carbon dioxide in the raw material gas may be 0.05 or less.
[4] In the production method according to any one of the above-mentioned items [1] to [3], a concentration of the carbon oxide in the raw material gas may be more than 9 vol% and 50 vol% or less.
[5] In the production method according to any one of the above-mentioned items [1] to [4], a concentration of the hydrogen in the raw material gas may be more than 44 vol% and 90 vol% or less.
[6] In the production method according to any one of the above-mentioned items [1] to [5], the raw material gas may have a temperature of 40°C or more and 350°C or less when caused to flow into the reactor.
[7] The production method according to any one of the above-mentioned items [1] to [6] may further include preparing the raw material gas by using a gas containing oxygen and carbon dioxide captured from air or a biogas.
[8] In the production method according to any one of the above-mentioned items [1] to [7], the reactor may include a water vapor separation membrane configured to cause water vapor to permeate therethrough.
[9] In the production method according to any one of the above-mentioned items [1] to [7], the reactor may include a liquid fuel separation membrane configured to cause at least the liquid fuel to permeate therethrough.
[10] According to an embodiment of the present invention, there is provided a liquid fuel synthesis system, including: a reactor storing a catalyst configured to advance a conversion reaction from a raw material gas containing a carbon oxide and hydrogen to a liquid fuel; and a raw material gas-supplying portion configured to supply, as the raw material gas, a gas, which contains the carbon oxide, the hydrogen, and oxygen, and in which a ratio of a concentration of carbon monoxide to a concentration of carbon dioxide in the carbon oxide is 0.9 or less, to the reactor.
[11] In the liquid fuel synthesis system according to the above-mentioned item [10], a concentration of the oxygen in the raw material gas may be 0.02 vol% or more and 4 vol% or less.
[12] In the liquid fuel synthesis system according to the above-mentioned item [10] or [11], a ratio of a concentration of the oxygen to a concentration of the carbon dioxide in the raw material gas may be 0.05 or less.
[13] The liquid fuel synthesis system according to any one of the above-mentioned items [10] to [12] may further include a gas-capturing portion configured to capture oxygen and carbon dioxide from air or a biogas, wherein a gas containing the oxygen and the carbon dioxide, which is to be supplied from the gas-capturing portion, is supplied as a constituent component for the raw material gas to the reactor.
[14] In the liquid fuel synthesis system according to any one of the above-mentioned items [10] to [13], the reactor may include a water vapor separation membrane configured to cause water vapor to permeate therethrough.
[15] In the liquid fuel synthesis system according to any one of the above-mentioned items [10] to [13], the reactor may include a liquid fuel separation membrane configured to cause at least the liquid fuel to permeate therethrough.

### Advantageous Effects of Invention

In the method of producing a liquid fuel according to the embodiment of the present invention, the gas containing the carbon oxide, hydrogen, and oxygen is used as the raw material gas. Thus, simultaneously with the production of the liquid fuel, water is produced from oxygen and hydrogen, and heat generated at the time is utilized to suppress a reduction in temperature near the raw material gas inflow port of the reactor. As a result, a reduction in reaction efficiency resulting from the reduction in temperature near the raw material gas inflow port can be suppressed. As related art, there exists the following autothermal reforming (ATR) method (e.g., JP 2004-43195 A): both of the partial oxidation reaction of a gas that is an exothermic reaction and the reforming reaction of the gas that is an endothermic reaction are sequentially caused in one reactor; and heat generated by the partial oxidation reaction is utilized in the reforming reaction. In the ATR method, the heat generation of the exothermic reaction compensates for the heat absorption of the endothermic reaction, and hence the effect of the method is exhibited on the downstream side of the reactor. In contrast, in the method of producing a liquid fuel according to the embodiment of the present invention, the heat generated by the exothermic reaction is utilized to suppress the reduction in temperature near the inlet of the reactor, thereby improving the reaction efficiency of the liquid fuel synthesis that is an exothermic reaction near the inlet of the reactor. Accordingly, the action and effect of the method are different from those of the ATR method.

### Brief Description of Drawings

FIG. 1 is a schematic view for describing a method of producing a liquid fuel according to one embodiment of the present invention.
FIG. **2** is a schematic view for describing the configuration of an example of a reactor that may be used in the method of producing a liquid fuel according to the embodiment of the present invention.
FIG. **3** is a schematic view for describing the configuration of an example of the reactor that may be used in the method of producing a liquid fuel according to the embodiment of the present invention.
FIGS. **4** are each a schematic view for describing the configuration of an example of the reactor that may be used in the method of producing a liquid fuel according to the embodiment of the present invention.
FIG. **5** is a schematic view for describing the configuration of an example of the reactor that may be used in the method of producing a liquid fuel according to the embodiment of the present invention.
FIG. **6** is a schematic view for describing a liquid fuel synthesis system to be used in the method of producing a liquid fuel according to one embodiment of the present invention.

### Description of Embodiments

Embodiments of the present invention are described below with reference to the drawings. However, the present invention is not limited to these embodiments. In addition, for clearer illustration, some widths, thicknesses, shapes, and the like of respective portions may be schematically illustrated in the drawings in comparison to the embodiments. However, the widths, the thicknesses, the shapes, and the like are each merely an example, and do not limit the understanding of the present invention.

### A. Method of producing Liquid Fuel

A method of producing a liquid fuel according to an embodiment of the present invention includes:
causing a raw material gas containing at least a carbon oxide and hydrogen to flow into a reactor storing a catalyst (step (I)); and
producing the liquid fuel from the raw material gas through a conversion reaction in the presence of the catalyst (step (II)).

Typically, the raw material gas further contains oxygen. The ratio of the concentration of carbon monoxide to the concentration of carbon dioxide in the carbon oxide is, for example, 0.9 or less.

The method of producing a liquid fuel according to the embodiment of the present invention typically further includes causing a product gas containing the above-mentioned liquid fuel to flow out of the above-mentioned reactor (step (III)).

In this description, the liquid fuel is a fuel that is in a liquid state at normal temperature and normal pressure or a fuel that can be liquefied under a normal-temperature pressurized state. Examples of the fuel that is in a liquid state at normal temperature and normal pressure include methanol, ethanol, a liquid fuel represented by CₙH₂₍ₘ₋₂ₙ₎ ("m" represents an integer of less than 90 and "n" represents an integer of less than 30), and a mixture thereof. Examples of the fuel that can be liquefied under a normal-temperature pressurized state include propane, butane, and a mixture thereof.

As an example of the above-mentioned conversion reaction, reactions that may occur at the time of the synthesis of methanol through the catalytic hydrogenation of a raw material gas containing carbon monoxide, carbon dioxide, and hydrogen in the presence of a catalyst are represented by the following reaction formulae. In the reaction represented by the formula (2), methanol serving as a product and water serving as a by-product are produced. With regard to a calorific value, a calorific value when the reaction proceeds to the right-hand side in each formula is described, and a negative sign represents heat generation and a positive sign represents heat absorption.

CO+2H₂ ↔ CH₃OH: -90.8 kJ/mol (1)

CO₂+3H₂ ↔ CH₃OH+H₂O: -49.2 kJ/mol (2)

CO+H₂O ↔ CO₂+H₂: -41.2 kJ/mol (3)

H₂+1/2O₂ ↔ H₂O: -241.8 kJ/mol (4)

CO+1/2O₂ ↔ CO₂: -283.0 kJ/mol (5)

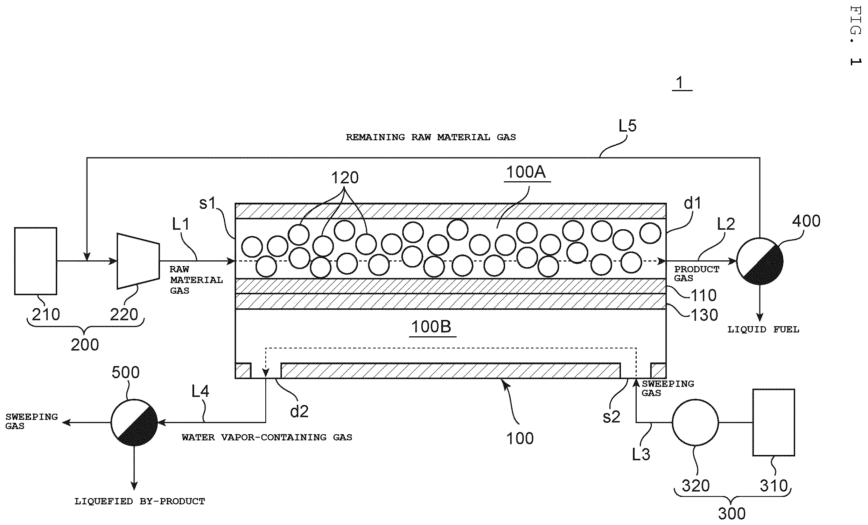

FIG. **1** is a schematic view for describing the method of producing a liquid fuel according to one embodiment of the present invention. The method of producing a liquid fuel according to the embodiment illustrated in FIG. **1** is performed by using a liquid fuel synthesis system **1** including: a reactor **100** storing a catalyst; and a raw material gas-supplying portion **200** configured to supply a raw material gas to the reactor **100.** The liquid fuel synthesis system **1** further includes a sweeping gas-supplying portion **300** that supplies a sweeping gas to the reactor **100.** The raw material gas-supplying portion **200** includes a first gas-storing portion **210** and a pressure-boosting portion **220.** The sweeping gas-supplying portion **300** includes a second gas-storing portion **310** and a heating portion **320.** The method of producing a liquid fuel according to the embodiment of the present invention is described below with reference to FIG. 1.

### A-1. Step (I)

In the step (I), the raw material gas containing the carbon oxide, hydrogen, and oxygen is caused to flow into the reactor storing the catalyst.

The reactor typically includes: a gas inflow port through which the raw material gas is caused to flow into the reactor; a gas outflow port through which the product gas containing the product is caused to flow out of the reactor; and a space that communicates the ports to each other. The catalyst is stored in the space.

The shape of the reactor is not particularly limited, and may be set to, for example, a monolith shape, a flat-plate shape, a tubular shape, a cylindrical shape, a columnar shape, or a polygonal columnar shape. The monolith shape means a shape having a plurality of cells penetrating the shape in its lengthwise direction, and is a concept including a honeycomb shape.

In the embodiment illustrated in FIG. **1****,** the reactor **100** is a so-called membrane reactor, and includes a water vapor separation membrane **110,** a catalyst **120,** a non-permeation-side space **100A,** and a permeation-side space **100B.** In the illustrated example, the water vapor separation membrane **110** is supported by a porous support **130.** The reactor **100** further includes: a first inflow port **s1** and a first outflow port **d1** communicating to each other through the non-permeation-side space **100A;** and a second inflow port **s2** and a second outflow port **d2** communicating to each other through the permeation-side space **100B.** In the reactor **100,** the non-permeation-side space **100A** is a space that stores the catalyst. The reactor **100** preferably has heat resistance and pressure resistance suitable for conditions for the synthesis of a desired liquid fuel.

The water vapor separation membrane **110** causes water vapor, which is a by-product of the conversion reaction from the raw material gas to the liquid fuel, to permeate therethrough. Thus, the reaction equilibrium of the formula (2) can be shifted to a product side through utilization of an equilibrium shift effect.

The molecular diameter (0.26 nm) of water is close to the molecular diameter (0.296 nm) of hydrogen. Accordingly, not only the water vapor that is a by-product of the conversion reaction but also part of hydrogen in the raw material gas can permeate through the water vapor separation membrane **110.**

The water vapor separation membrane **110** preferably has a water vapor permeance of 100 nmol/(s·Pa·m²) or more. The water vapor permeance may be determined by an existing method (see Ind. Eng. Chem. Res., 40, 163-175 (2001)).

The water vapor separation membrane **110** preferably has a separation factor of 100 or more. As the separation factor becomes larger, the membrane more easily causes the water vapor to permeate therethrough, and more hardly causes components except the water vapor (e.g., hydrogen, the carbon oxide, oxygen, and the liquid fuel) to permeate therethrough. The separation factor may be determined by an existing method (see Fig. 1 of "Separation and Purification Technology 239 (2020) 116533").

An inorganic membrane may be used as the water vapor separation membrane **110.** The inorganic membrane is preferred because the membrane has heat resistance, pressure resistance, and water vapor resistance. Examples of the inorganic membrane include a zeolite membrane, a silica membrane, an alumina membrane, and a composite membrane thereof. For example, a LTA zeolite membrane in which the molar ratio (Si/Al) of a silicon element (Si) to an aluminum element (Al) is 1.0 or more and 3.0 or less is suitable because the membrane is excellent in water vapor permeability.

The zeolite membrane to be used as the water vapor separation membrane **110** may be obtained by a production method described in, for example, JP 2004-66188 A. In addition, the silica membrane to be used as the water vapor separation membrane **110** may be obtained by a production method described in, for example, WO 2008/050812 A1.

The porous support **130** includes a porous material. For example, a ceramic material, a metal material, a resin material, and a composite member thereof may each be used as the porous material, and the ceramic material is particularly suitable. For example, alumina (Al₂O₃), titania (TiO₂), mullite (Al₂O₃·SiO₂), ceramic fragments, cordierite (Mg₂Al₄Si₅O₁₈), and a composite material containing two or more of these materials may each be used as an aggregate for the ceramic material, and alumina is suitable in consideration of ease of availability, green body stability, and corrosion resistance. At least one of titania, mullite, easily sinterable alumina, silica, glass frit, a clay mineral, or easily sinterable cordierite may be used as an inorganic binder for the ceramic material. The ceramic material may be free of any inorganic binder.

The average pore diameter of the porous support may be set to 5 µm or more and 25 µm or less. The average pore diameter of the porous support may be measured by a mercury intrusion method. The porosity of the porous support may be set to 25% or more and 50% or less. The average particle diameter of the porous material for forming the porous support may be set to 1 µm or more and 100 µm or less. In this embodiment, the average particle diameter is the arithmetic average value of the maximum diameters of 30 measurement object particles (randomly selected) measured by sectional microstructure observation with a scanning electron microscope (SEM).

The catalyst **120** advances the conversion reaction from the raw material gas to the liquid fuel. The catalyst is arranged in the non-permeation-side space **100A.** Although the catalyst is preferably filled into the non-permeation-side space **100A,** the catalyst may be arranged in a layer shape or an island shape on the surface of the water vapor separation membrane **110.** When the catalyst has a particle shape like the illustrated example, the particle diameters (diameters) of catalyst particles may each be set to, for example, 0.5 mm or more and 10 mm or less. Each of the catalyst particles may be formed only of the catalyst, or may have a configuration in which the catalyst is carried by a carrier particle. The carrier particle is preferably a porous particle.

Any catalyst suitable for a conversion reaction to a desired liquid fuel may be used as the catalyst. Specifically, metal catalysts (e.g., copper and palladium), oxide catalysts (e.g., zinc oxide, zirconia, and gallium oxide), and catalysts obtained by compositing these catalysts (e.g., copper-zinc oxide, copper-zinc oxide-alumina, copper-zinc oxide-chromium oxide-alumina, copper-cobalt-titania, and catalysts obtained by modifying these catalysts with palladium) may each be used.

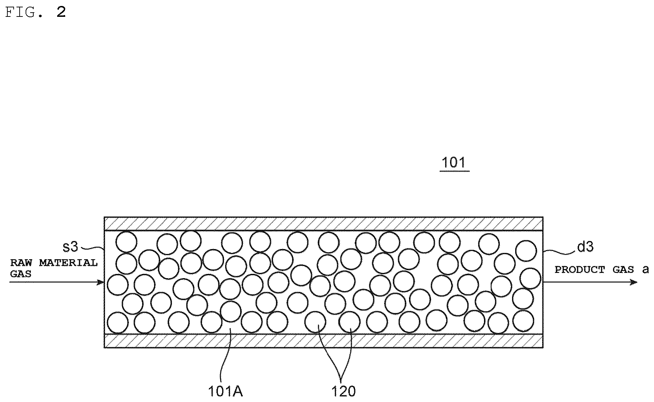

The reactor is not limited to the above-mentioned illustrated example. For example, as illustrated in FIG. **2****,** a reactor **101** may be a flow reactor including: a gas inflow port **s3;** a gas outflow port **d3;** a space **101A** that communicates the ports to each other; and the catalyst (catalyst particles) **120** arranged (filled) in the space **101A.** The raw material gas containing the carbon oxide, hydrogen, and oxygen is caused to flow into the reactor **101** through the gas inflow port **s3,** and a product gas (product gas **"a")** containing the liquid fuel, a by-product, and an unreacted raw material gas (remaining raw material gas) is caused to flow out of the reactor through the gas outflow port **d3.**

As described above, the raw material gas contains the carbon oxide, hydrogen, and oxygen. At least carbon dioxide is used as the carbon oxide, and carbon monoxide may be further used to the extent that the effect of the present invention is obtained. The raw material gas preferably contains carbon dioxide at a ratio higher than that of carbon monoxide. When the ratio (CO vol%/CO₂ vol%, hereinafter sometimes referred to as "CO/CO₂ ratio") of the concentration of carbon monoxide to the concentration of carbon dioxide in the carbon oxide in the raw material gas is large, the effect of the present invention exhibited by the blending of oxygen may not be sufficiently obtained. Specifically, when the above-mentioned CO/CO₂ ratio is large, the suppressing effect of the blending of oxygen on a reduction in temperature near the raw material gas inflow port of the reactor weakens because of the following reasons: when the reaction represented by the formula (1) and the reaction represented by the formula (2) are compared to each other, the reaction represented by the formula (1) has the larger calorific value; and the equilibrium conversion ratio of the reaction represented by the formula (1) is larger than that of the reaction represented by the formula (2), and hence the calorific value further increases. The above-mentioned CO/CO₂ ratio is typically 0.9 or less. In this case, as the inlet temperature of the reactor reduces, the equilibrium conversion ratio increases, and hence a temperature change due to heat generation becomes larger. Accordingly, from the viewpoints of the equilibrium conversion ratio and temperature unevenness, when the reactor is operated at an inlet temperature of 250°C or less, the CO/CO₂ ratio is preferably 0.5 or less, and when the reactor is operated at an inlet temperature of 230°C or less, the CO/CO₂ ratio is preferably 0.2 or less. The lower limit value of the CO/CO₂ ratio is not particularly limited because after the passage of the catalyst, the reaction represented by the formula (3) occurs. However, the CO/CO₂ ratio is preferably set to 0.02 or more from the viewpoint of the ease with which the ratio is controlled. The above-mentioned CO/CO₂ ratio is a CO/CO₂ ratio in the raw material gas at the inlet (specifically, the first inflow port **s1)** of the reactor, and when the remaining raw material gas is recycled, the ratio is a CO/CO₂ ratio in a raw material gas prepared by using the remaining raw material gas. In addition, other descriptions concerning, for example, the contents and ratios of the respective gases to be incorporated into the raw material gas are descriptions about the raw material gas at the inlet of the reactor.

The concentration of the carbon oxide in the raw material gas (when the gas contains carbon monoxide and carbon dioxide, their total concentration) may be more than 9 vol% and 50 vol% or less, and is, for example, 10 vol% or more and 50 vol% or less, preferably 20 vol% or more and 40 vol% or less. In one embodiment, the concentration of carbon dioxide in the raw material gas is, for example, 10 vol% or more and 50 vol% or less, preferably 15 vol% or more and 35 vol% or less. In one embodiment, the concentration of carbon monoxide in the raw material gas is, for example, 23 vol% or less, preferably 20 vol% or less. The concentration of hydrogen in the raw material gas may be more than 44 vol% and 90 vol% or less, and is, for example, 45 vol% or more and 90 vol% or less, preferably 60 vol% or more and 80 vol% or less.

The concentration of oxygen in the raw material gas is, for example, 0.02 vol% or more, preferably 0.03 vol% or more, more preferably 0.1 vol% or more, and is, for example, 4 vol% or less, preferably 3 vol% or less, more preferably 2 vol% or less. When the concentration of oxygen is less than 0.02 vol%, the effect of the present invention may not be sufficiently obtained. When the concentration of oxygen is more than 4 vol%, hydrogen that should have originally reacted with carbon dioxide may be consumed to reduce the reaction yield of the liquid fuel, or a reaction temperature may increase more than expected to reduce the equilibrium conversion ratio, thereby reducing the reaction yield. As described above, when oxygen is present in the raw material gas, the following is assumed: hydrogen is consumed by a reaction between oxygen and hydrogen without contributing to the production of the liquid fuel; and water produced by the reaction shifts the reaction equilibrium of the formula (2) to a raw material gas side to reduce the reaction yield. However, according to the embodiment of the present invention, contrary to those assumptions, the utilization of the reaction heat of the reaction can provide a suppressing effect on a reduction in reaction efficiency. In one embodiment, the ratio (O₂ vol%/CO₂ vol%, hereinafter sometimes referred to as "O₂/CO₂ ratio") of the concentration of oxygen to the concentration of carbon dioxide in the raw material gas is, for example, 0.05 or less. When the O₂/CO₂ ratio increases, in addition to the reduction in reaction yield due to the consumption of hydrogen serving as a raw material, the equilibrium conversion ratio may reduce along with an increase in calorific value. For example, when the reactor is operated at an inlet temperature of 250°C or less, the O₂/CO₂ ratio is preferably set to 0.02 or less. In addition, after the passage of the catalyst, the reaction represented by the formula (5) occurs. Accordingly, from the viewpoint of controlling a detector or a mixing valve, the O₂/CO₂ ratio is preferably set to 0.0015 or more because the detector or the valve is easily controlled for stably operating the reactor.

The total concentration of the carbon oxide, hydrogen, and oxygen in the raw material gas is, for example, 80 vol% or more and 100 vol% or less, preferably 96 vol% or more and 100 vol% or less. The raw material gas may be formed substantially only of the carbon oxide, hydrogen, and oxygen.

The raw material gas is prepared by mixing the respective components so that desired composition may be obtained. In one embodiment, the raw material gas is prepared by using a carbon dioxide-enriched gas captured from air by direct air capture (DAC). The carbon dioxide-enriched gas obtained by the DAC contains oxygen and carbon dioxide captured from the air. Specifically, the carbon dioxide-enriched gas obtained by the DAC may contain a trace amount (e.g., 0.02 vol% or more and 15 vol% or less) of oxygen derived from the air in addition to a high concentration (e.g., 30 vol% or more and 99.9 vol% or less) of carbon dioxide. In addition, the concentration of carbon monoxide in the carbon dioxide-enriched gas is low. Accordingly, when the raw material gas is prepared by using the carbon dioxide-enriched gas obtained by the DAC, the following advantage is obtained: there is no need to separately prepare and mix oxygen; or the amount of oxygen to be separately prepared can be reduced. In another embodiment, the raw material gas is prepared by using a carbon dioxide-enriched gas captured from a biogas. The biogas is a gas produced through fermentation (methane fermentation) by using biomass, such as garbage, waste paper, or livestock manure, as a raw material. The main components of the biogas are methane and carbon dioxide, and the biogas may further contain oxygen or the like. The use of a separation membrane can capture carbon dioxide from the biogas to provide the carbon dioxide-enriched gas. The carbon dioxide-enriched gas captured from the biogas may contain oxygen in addition to carbon dioxide, and the concentration of carbon monoxide therein is low. Accordingly, when the raw material gas is prepared by using the carbon dioxide-enriched gas captured from the biogas, the following advantage is obtained: there is no need to separately prepare and mix oxygen; or the amount of oxygen to be separately prepared can be reduced. In one embodiment, the liquid fuel synthesis system **1** may be configured as follows: the system further includes a gas-capturing portion configured to capture oxygen and carbon dioxide from the air through the DAC, or a gas-capturing portion configured to capture oxygen and carbon dioxide from the biogas with the separation membrane, and the carbon dioxide-enriched gas (gas containing oxygen and carbon dioxide captured from the air or the biogas) is supplied from the gas-capturing portion to the raw material gas-supplying portion. The carbon dioxide-enriched gas may be directly supplied from the gas-capturing portion to the raw material gas-supplying portion, or may be supplied to the raw material gas-supplying portion after having passed as a constituent component for a sweeping gas through the reactor. For example, the gas-capturing portion includes a carbon dioxide adsorbent (e.g., an amine compound), which adsorbs carbon dioxide by being brought into contact with a carbon dioxide-containing gas, and which desorbs carbon dioxide through heating, a pressure reduction, or the like, and the portion can capture a gas containing oxygen and a high concentration of carbon dioxide from the air with the carbon dioxide adsorbent. In addition, for example, the gas-capturing portion includes a separation membrane such as a carbon dioxide-permeable membrane, and can capture a carbon dioxide-enriched gas containing oxygen and a high concentration of carbon dioxide from the carbon dioxide-containing gas such as a biogas.

The raw material gas is flowed into the reactor at a desired temperature and a desired pressure. In the embodiment illustrated in FIG. **1****,** the gas containing the carbon oxide, hydrogen, and oxygen, the gas being stored in the first gas-storing portion **210,** is mixed with the remaining raw material gas, and the pressure and temperature of the mixture are increased in the pressure-boosting portion **220.** After that, the mixture is flowed as the raw material gas into the non-permeation-side space **100A** of the reactor **100** through a line **L1.** The pressure-boosting portion **220** includes, for example, a compressor or a pressure booster. In the illustrated example, the raw material gas prepared in desired composition in advance is supplied from the first gas-storing portion **210** to the reactor. However, the raw material gas only needs to be flowed in the desired composition into the reactor, and hence the time point at which the desired composition is achieved is not limited to the embodiment of the illustrated example. For example, the carbon oxide, hydrogen, and oxygen may be separately caused to flow from their respective supply sources into the reactor.

The temperature of the raw material gas when the gas is caused to flow into the reactor (in other words, the temperature of the raw material gas at the gas inflow port of the reactor) is, for example, 40°C or more and 350°C or less, preferably 180°C or more and 330°C or less.

The pressure of the raw material gas when the gas is caused to flow into the reactor (in other words, the pressure thereof at the gas inflow port of the reactor) is, for example, 1.0 MPa (G) or more and 6.0 MPa (G) or less, preferably 2.0 MPa (G) or more and 6.0 MPa (G) or less.

The flow rate of the raw material gas to be caused to flow into the reactor is, for example, 1,000/h or more and 50,000/h or less, preferably 2,000/h or more and 20,000/h or less, more preferably 3,000/h or more and 12,000/h or less in terms of space velocity GHSV.

### A-2. Step (II)

In the step (II), in the presence of the catalyst, the liquid fuel is produced from the raw material gas by the conversion reaction. As represented by, for example, the reaction formulae (1) and (2), the carbon oxide and hydrogen are subjected to catalytic hydrogenation in the presence of the catalyst to produce methanol. Each of the produced liquid fuel and a by-product (typically, water) is in a gas state at the time of its synthesis, and at least until the liquid fuel or the by-product flows out of the reactor, the liquid fuel or the by-product is maintained while being in a gas state.

According to the embodiment illustrated in FIG. **1****,** the raw material gas passes through the first inflow port **s1** to flow into the non-permeation-side space **100A,** and the catalyst **120** advances the conversion reaction from the raw material gas to the liquid fuel. Thus, the liquid fuel is synthesized. In addition, water vapor that is a by-product and hydrogen in the raw material gas permeate through the water vapor separation membrane **110** to flow into the permeation-side space **100B.** The sweeping gas for sweeping the water vapor and hydrogen is flowed from the second inflow port **s2** into the permeation-side space **100B** through a line **L3.** Specifically, the sweeping gas stored in the second gas-storing portion **310** is heated to a desired temperature (e.g., 150°C or more and 350°C or less) in the heating portion **320,** and is then flowed into the permeation-side space **100B** of the reactor **100** through the line **L3.** Any appropriate gases, such as nitrogen, a carbon oxide, hydrogen, and a mixed gas thereof, may each be used as the sweeping gas.

Each of the reactions represented by the reaction formulae (1) to (5) is an equilibrium reaction, and hence, to increase both of its conversion ratio and reaction rate, the reaction is preferably performed under high temperature and high pressure. The reaction temperature (in other words, the temperature of the space of the reactor having stored therein the catalyst) is, for example, 180°C or more, preferably 200°C or more and 350°C or less, more preferably 200°C or more and 300°C or less. The reaction pressure (in other words, the pressure of the space of the reactor having stored therein the catalyst) is, for example, 1 MPa (G) or more, preferably 2.0 MPa (G) or more and 6.0 MPa (G) or less, more preferably 2.5 MPa (G) or more and 4.0 MPa (G) or less.

In each of the reaction formulae (1) and (2), the production of methanol is an exothermic reaction, and hence generated heat is effective in maintaining its reaction temperature. However, near the gas inflow port of the reactor, the generated heat is flowed toward the downstream side thereof by a gas flow, and hence a sufficiently high reaction temperature is not obtained in some cases. In contrast, according to the embodiment of the present invention, the raw material gas contains oxygen, and hence water is produced from oxygen and hydrogen in the presence of the above-mentioned catalyst. In the production of the water, a calorific value is larger than that in the production of methanol. Accordingly, even when the heat generated near the gas inflow port is flowed toward the downstream side by the gas flow, a temperature near the gas inflow port can be increased as compared to that in an embodiment in which the raw material gas is free of oxygen. As a result, the following effect can be obtained: a reduction in reaction efficiency near the gas inflow port is suppressed.

### A-3. Step (III)

In the step (III), the product gas containing the liquid fuel is caused to flow out of the reactor. The product gas typically contains the liquid fuel and the remaining raw material gas. Accordingly, the liquid fuel is preferably separated and recovered from the product gas.

The temperature of the product gas when the gas is caused to flow out of the reactor (in other words, the temperature of the product gas at the gas outflow port of the reactor) is, for example, 200°C or more and 350°C or less, preferably 200°C or more and 300°C or less.

In the embodiment illustrated in FIG. **1****,** the product gas passes through the first outflow port **d1** to be flowed out of the reactor **100** (more specifically, the non-permeation-side space **100A)** into a line **L2.** In addition, the water vapor and hydrogen that have permeated through the water vapor separation membrane **110** to flow into the permeation-side space **100B** pass through the second outflow port **d2** together with the sweeping gas to be flowed as a water vapor-containing gas out of the reactor **100** (more specifically, the permeation-side space **100B)** into a line **L4.**

The product gas that has been flowed out of the reactor **100** is flowed into a drain trap **400** arranged downstream of the reactor **100.** The drain trap **400** liquefies the liquid fuel in a gas state, and separates the remaining raw material gas from the liquid fuel. Thus, the liquid fuel is recovered. Meanwhile, the remaining raw material gas thus separated is returned to the upstream of the reactor by a line **L5,** and is mixed with the gas that has been flowed out of the first gas-storing portion. Thus, the raw material gas is obtained. From the viewpoint of adjusting the composition of the raw material gas, the remaining raw material gas may be purged as required. The recycling rate of the remaining raw material gas in the raw material gas (volume percentage of the remaining raw material gas in the raw material gas) may be, for example, 15% or more and 85% or less. The resultant raw material gas is subjected to a pressure increase and a temperature increase in the pressure-boosting portion **220,** and is flowed into the reactor **100** through the line **L1.** As described above, in the embodiment of the present invention, the concentration ratios of the respective gases in the raw material gas are suitably controlled, and hence even when the remaining raw material gas is recycled, the liquid fuel can be continuously produced while the effect of the present invention is maintained.

With regard to the water vapor-containing gas that has been caused to flow out of the reactor **100,** similarly, a condensable by-product (typically, water vapor) is liquefied, and is separated from the sweeping gas and hydrogen and captured by a drain trap **500.** The following may be performed as required: hydrogen or the like is recovered from the separated gas, and is reused as part of the raw material gas.

### B. Modification Examples

The present invention is not limited to the above-mentioned embodiments, and various modifications may be made thereto. For example, the configurations described in the above-mentioned embodiments may each be replaced by substantially the same configuration, a configuration having the same action and effect, and a configuration that can achieve the same object.

### B-1. Modification Example 1

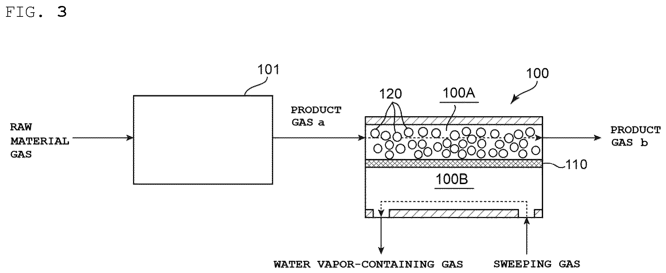

The reactor may be obtained by combining a plurality of reactors. For example, as illustrated in FIG. **3****,** the reactor may have a configuration in which the flow reactor **101** illustrated in FIG. **2** is arranged upstream of the reactor **100** that is a membrane reactor. The reactor **100** includes the water vapor separation membrane **110,** the catalyst **120,** the non-permeation-side space **100A,** and the permeation-side space **100B.** The water vapor separation membrane **110** is typically supported by a porous support (not shown). According to the reactor having such configuration, the product gas **"a"** that has flowed out of the reactor **101,** the gas containing the liquid fuel, the water vapor, and the remaining raw material gas, flows into the non-permeation-side space **100A** of the reactor **100,** and hence a conversion reaction from the remaining raw material gas to the liquid fuel proceeds. A product gas **"b"** containing the liquid fuel produced in the reactors **101** and **100** flows out of the non-permeation-side space **100A.** The water vapor that has permeated through the water vapor separation membrane **110** and the sweeping gas flow as a water vapor-containing gas out of the permeation-side space **100B.**

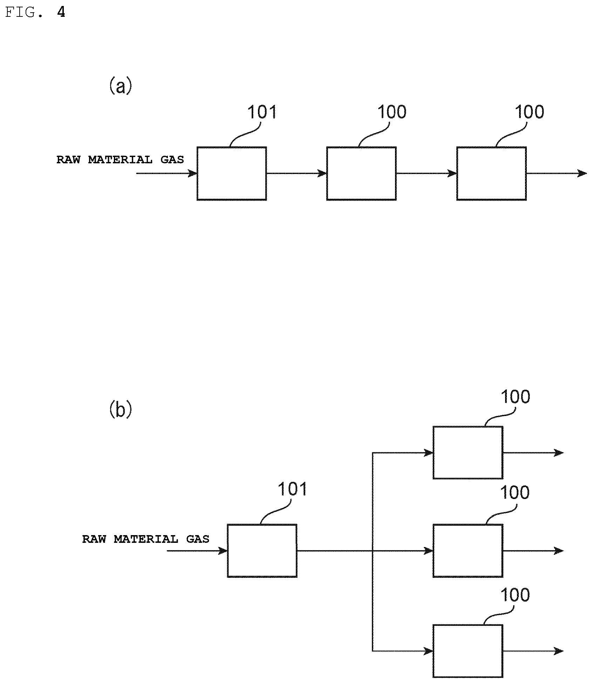

The number of the reactors **100** to be arranged downstream of the flow reactor **101** is not limited, and as illustrated in each of FIGS. **4****,** the two or more reactors **100** may be connected in series and/or in parallel. In each of the figures, with regard to the downstream of the reactor **101,** only the flow of the product gas is illustrated.

### B-2. Modification Example 2

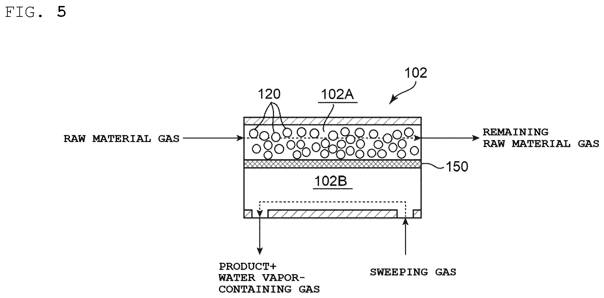

The reactor of the above-mentioned embodiment has the following configuration: the water vapor separation membrane that causes water vapor to permeate therethrough is used as a separation membrane, which separates the water vapor and the liquid fuel from each other, to cause the water vapor to permeate from the non-permeation-side space to the permeation-side space, thereby separating the water vapor. However, the reactor may have the following configuration: a liquid fuel separation membrane that causes the liquid fuel to permeate therethrough is used to cause the liquid fuel to permeate from the non-permeation-side space to the permeation-side space, thereby separating the liquid fuel. For example, a reactor **102** illustrated in FIG. **5** includes a liquid fuel separation membrane **150,** the catalyst **120,** a non-permeation-side space **102A,** and a permeation-side space **102B.** The liquid fuel separation membrane **150** may be typically supported by a porous support (not shown). According to such configuration, the liquid fuel permeates from the non-permeation-side space **102A** to the permeation-side space **102B,** and hence the reaction equilibrium of each of the formulae (1) and (2) can be shifted to a product side. A membrane described in JP 2020-23488 A may be used as a separation membrane that selectively causes the liquid fuel to permeate therethrough. In the present invention, the separation membrane that separates the water vapor and the liquid fuel from each other has higher selective permeability for one of the water vapor and the liquid fuel than for the other thereof, and may not completely separate both the water vapor and the liquid fuel from each other as long as the effect of the present invention is obtained. For example, the liquid fuel separation membrane causes the liquid fuel to permeate therethrough with selectivity higher than that of the water vapor, and does not completely separate both the water vapor and the liquid fuel from each other.

### B-3. Modification Example 3

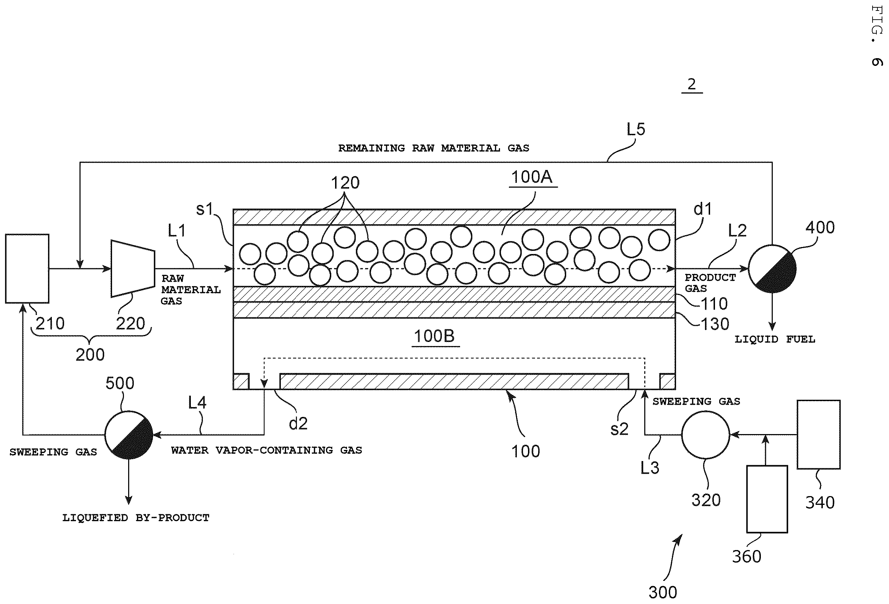

As described above, the raw material gas may be prepared by using a carbon dioxide-enriched gas captured from air by the DAC or from a biogas with a separation membrane (e.g., a gas containing oxygen and carbon dioxide captured from the air or from the biogas). Accordingly, the liquid fuel synthesis system to be used in the method of producing a liquid fuel according to the embodiment of the present invention may be configured as follows: the system further includes a gas-capturing portion configured to capture oxygen and carbon dioxide from the air by the DAC, or from the biogas with the separation membrane, and the carbon dioxide-enriched gas to be supplied from the gas-capturing portion is supplied as a constituent component for the raw material gas to the reactor. For example, like a liquid fuel synthesis system **2** illustrated in FIG. **6****,** the following configuration is permitted: the sweeping gas-supplying portion **300** includes a gas-capturing portion **340** and a hydrogen-producing portion **360,** and a sweeping gas prepared by mixing the carbon dioxide-enriched gas supplied from the gas-capturing portion **340** and hydrogen supplied from the hydrogen-producing portion **360** is supplied as a constituent component for the raw material gas to the reactor **100.** In Modification Example 3, the total amount of carbon dioxide to be incorporated into the raw material gas may be derived from the carbon dioxide-enriched gas captured in the gas-capturing portion **340,** or only part thereof may be derived from the carbon dioxide-enriched gas.

The gas-capturing portion **340** typically includes a carbon dioxide adsorbent (e.g., an amine compound), which adsorbs carbon dioxide by being brought into contact with a carbon dioxide-containing gas, and which desorbs carbon dioxide through heating, a pressure reduction, or the like, and the portion can capture a carbon dioxide-enriched gas containing oxygen and a high concentration of carbon dioxide from the air with the carbon dioxide adsorbent. Alternatively, the gas-capturing portion **340** includes a separation membrane such as a carbon dioxide-permeable membrane, and can capture a carbon dioxide-enriched gas containing oxygen and a high concentration of carbon dioxide from the carbon dioxide-containing gas such as a biogas. The hydrogen-producing portion **360** is, for example, a hydrogen-producing facility utilizing a water electrolysis technology.

### Industrial Applicability

The method of producing a liquid fuel according to the embodiment of the present invention may be suitably used in the production of a liquid fuel such as methanol.

### Reference Signs List

**100** reactor
**100A** non-permeation-side space
**100B** permeation-side space
**110** water vapor separation membrane
**120** catalyst
**200** raw material gas-supplying portion
**300** sweeping gas-supplying portion

## Claims

1. A method of producing a liquid fuel, comprising:
causing a raw material gas containing at least a carbon oxide and hydrogen to flow into a reactor storing a catalyst; and
producing the liquid fuel from the raw material gas through a conversion reaction in the presence of the catalyst,
wherein the raw material gas further contains oxygen, and
wherein a ratio of a concentration of carbon monoxide to a concentration of carbon dioxide in the carbon oxide is 0.9 or less.

2. The production method according to claim 1, wherein a concentration of the oxygen in the raw material gas is 0.02 vol% or more and 4 vol% or less.

3. The production method according to claim 1, wherein a ratio of a concentration of the oxygen to a concentration of the carbon dioxide in the raw material gas is 0.05 or less.

4. The production method according to claim 1, wherein a concentration of the carbon oxide in the raw material gas is more than 9 vol% and 50 vol% or less.

5. The production method according to claim 1, wherein a concentration of the hydrogen in the raw material gas is more than 44 vol% and 90 vol% or less.

6. The production method according to claim 1, wherein the raw material gas has a temperature of 40°C or more and 350°C or less when caused to flow into the reactor.

7. The production method according to claim 1, further comprising preparing the raw material gas by using a gas containing oxygen and carbon dioxide captured from air or a biogas.

8. The production method according to any one of claims 1 to 7, wherein the reactor includes a water vapor separation membrane configured to cause water vapor to permeate therethrough.

9. The production method according to any one of claims 1 to 7, wherein the reactor includes a liquid fuel separation membrane configured to cause at least the liquid fuel to permeate therethrough.

10. A liquid fuel synthesis system, comprising:
a reactor storing a catalyst configured to advance a conversion reaction from a raw material gas containing at least a carbon oxide and hydrogen to a liquid fuel; and
a raw material gas-supplying portion configured to supply, as the raw material gas, a gas, which contains the carbon oxide, the hydrogen, and oxygen, and in which a ratio of a concentration of carbon monoxide to a concentration of carbon dioxide in the carbon oxide is 0.9 or less, to the reactor.

11. The liquid fuel synthesis system according to claim 10, wherein a concentration of the oxygen in the raw material gas is 0.02 vol% or more and 4 vol% or less.

12. The liquid fuel synthesis system according to claim 10, wherein a ratio of a concentration of the oxygen to a concentration of the carbon dioxide in the raw material gas is 0.05 or less.

13. The liquid fuel synthesis system according to claim 10, further comprising a gas-capturing portion configured to capture oxygen and carbon dioxide from air or a biogas,
wherein a gas containing the oxygen and the carbon dioxide, which is to be supplied from the gas-capturing portion, is supplied as a constituent component for the raw material gas to the reactor.

14. The liquid fuel synthesis system according to any one of claims 10 to 13, wherein the reactor includes a water vapor separation membrane configured to cause water vapor to permeate therethrough.

15. The liquid fuel synthesis system according to any one of claims 10 to 13, wherein the reactor includes a liquid fuel separation membrane configured to cause at least the liquid fuel to permeate therethrough.
